# EUROPÄISCHE PATENTSCHRIFT

(11) **EP 0 568 977 B1**
(45) Veröffentlichungstag und Bekanntmachung des Hinweises auf die Patenterteilung: **21.08.1996**
(21) Anmeldenummer: 93107214.4
(22) Anmeldetag: 04.05.1993
(51) Int. Cl.: C07C 209/90, C07C 211/45

(54) **Stabilisierte Aminobenzotrifluoride**
Stabilized aminobenzotrifluorides
Aminobenzotrifluorides stabilisés

(30) Priorität: 05.05.1992 DE 4214849
(43) Veröffentlichungstag der Anmeldung: 10.11.1993
(73) Patentinhaber: HOECHST AKTIENGESELLSCHAFT, 65926 Frankfurt am Main (DE)
(72) Erfinder: Billeb, Gilbert, Dr., W-6233 Kelkheim/Ts. (DE); Semler, Günther, Dr., W-6233 Kelkheim/Ts. (DE)

(56) Entgegenhaltungen:
- EP-A- 0 398 542
- US-A- 2 911 340
- US-A- 3 324 011
- JOURNAL OF THE AMERICAN CHEMICAL SOCIETY, Bd. 69, 1947, Washington, DC, US, Seiten 2346 - 2350, R. G. JONES: "Ortho and Para Substituted Derivatives of Benzotrifluoride"

## Beschreibung

Aminobenzotrifluoride sind als Ausgangsprodukte zur Herstellung zahlreicher relevanter Verbindungen beispielsweise auf dem Gebiet der Pflanzenschutzmittel und Pharmazeutika von großer technischer Bedeutung.

Aminobenzotrifluoride besitzen geringe Hitze- und Lagerstabilität. In R.G. Jones, J. Amer. Chem. Soc. 69, 2346 (1947) ist beschrieben, daß 2- und 4-Aminobenzotrifluorid beim Erhitzen in glasartige Polymere übergehen.

2-Aminobenzotrifluorid verharzt bei 140 bis 150°C in weniger als 2 Stunden. 4-Aminobenzotrifluorid ist sogar bereits bei 120°C nicht mehr stabil und verharzt innerhalb von 1,5 Stunden. Chlorsubstituenten am Ring erhöhen zwar die Stabilität, jedoch tritt auch hier ab etwa 180°C eine Verharzung ein. Diese Eigenschaft erschwert die Reindarstellung dieser Verbindungen, beispielsweise durch Destillation, erheblich, da die Siedepunkte selbst bei vermindertem Druck der Zersetzungstemperatur sehr nahe kommen. Die Destillate sind oftmals instabiler als die Rohprodukte. Auch für die Lagerung dieser Verbindungen, beispielsweise als Schmelzen, müssen Vorkehrungen gegen Überhitzung getroffen werden, zumal die Verharzung exotherm abläuft.

Der vorliegenden Erfindung lag daher die Aufgabe zugrunde, Aminobenzotrifluoride in einer stabilisierten Form zur Verfügung zu stellen, in der sie die vorstehend geschilderten Nachteile überwinden.

Es wurde gefunden, daß man Aminobenzotrifluoride überraschenderweise gegen thermische Zersetzung und Verharzung stabilisieren kann, wenn man ihnen eine basische Verbindung zusetzt.

Gegenstand der vorliegenden Erfindung sind Präparationen eines stabilisierten Aminobenzotrifluorids, bestehend im wesentlichen aus
a) einem Aminobenzotrifluorid der Formel (I), 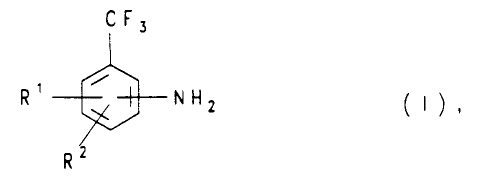 worin
   R¹ und R² unabhängig voneinander Wasserstoff, C₁-C₆-Alkyl, vorzugsweise C₁-C₄-Alkyl, insbesondere Methyl; C₂-C₆-Alkenyl, vorzugsweise C₂-C₄-Alkenyl; substituiertes oder unsubstituiertes Aryl, vorzugsweise unsubstituiertes oder substituiertes Phenyl, insbesondere Phenyl, Hydroxyphenyl, Tolyl, Xylyl, Aminophenyl, Halogenphenyl und Nitrophenyl; Halogen, vorzugsweise Fluor, Chlor oder Brom; CF₃, Nitro, Amino, Methoxy, Hydroxy oder -NR³R⁴ bedeuten, wobei R³ und R⁴ unabhängig voneinander für Wasserstoff oder C₁-C₅-Alkylreste, vorzugsweise Wasserstoff oder C₁-C₃-Alkylreste, stehen, und die NH₂-Gruppe in 2-, 3- oder 4-Position zur CF₃-Gruppe stehen kann und
b) mindestens einer Base.

Als Basen werden erfindungsgemäß vorzugsweise primäre, sekundäre oder tertiäre aliphatische oder araliphatische Amine der Formeln (IIa) oder (IIb) oder cycloaliphatische Amine der Formel (IIc) eingesetzt

R⁵R⁶R⁷N (IIa),

R⁸-[-NR⁹-(CR¹³R¹⁴)ₙ-]ₘ-NR¹⁰R¹¹ (IIb),

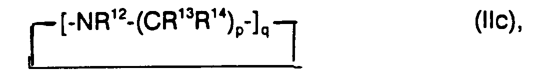

worin
- R⁵, R⁶ und R⁷: unabhängig voneinander jeweils für Wasserstoff, C₁-C₄₀-Alkyl, vorzugsweise C₁-C₂₀-Alkyl, C₃-C₈-Cycloalkyl, vorzugsweise C₅-C₆-Cycloalkyl, CY¹Y²-Aryl, wobei Y¹ und Y² unabhängig voneinander C₁-C₄-Alkyl, Aryl oder Wasserstoff bedeuten und Aryl vorzugsweise für substituiertes oder unsubstituiertes Phenyl, insbesondere für Phenyl, Aminophenyl, Hydroxyphenyl, Tolyl, Xylyl, Nitrophenyl und Halogenphenyl, steht,
- R⁸, R⁹, R¹⁰, R¹¹ und R¹²: jeweils unabhängig voneinander für Wasserstoff, C₁-C₂₀-Alkyl oder C₃-C₈-Cycloalkyl, vorzugsweise für Wasserstoff oder C₁-C₃-Alkyl, stehen, wobei R¹² zusätzlich die Bedeutung -(CR¹³R¹⁴)ₚ- haben kann,
- R¹³ und R¹⁴: unabhängig voneinander für Wasserstoff oder Methyl stehen,
- m: für eine ganze Zahl von 1 bis 5, vorzugsweise 1 bis 2,
- n: für eine ganze Zahl von 2 bis 10, vorzugsweise 2 bis 3,
- p: für eine ganze Zahl von 1 bis 8, vorzugsweise 1 bis 6, und
- q: für eine ganze Zahl von 1 bis 5, vorzugsweise 1 bis 4, stehen.

Bevorzugte Basen im Sinne der vorliegenden Erfindung sind ferner Alkalicarbonate, Erdalkalicarbonate, Alkalihydrogencarbonate, Alkalihydrogenphosphate, Erdalkalihydrogenphosphate, Alkalialkoholate, Erdalkalialkoholate, Alkalihydroxide oder Erdalkalihydroxide, wobei Alkali vorzugsweise für Lithium, Natrium oder Kalium und Erdalkali vorzugsweise für Magnesium oder Calcium stehen, oder eine Mischung aus mindestens zwei der vorstehend genannten Basen.

Von den Aminen werden zum Zweck der vorliegenden Erfindung vorzugsweise solche ausgewählt, deren Siedepunkte hoch genug liegen, daß sie bei einer Destillation im Sumpf verbleiben, obwohl dies nicht zwingend notwendig ist. Niedrigsiedende Amine kann man auch nach Bedarf oder gegebenenfalls kontinuierlich nachdosieren.

Von den Aminen der Formel (IIa) werden erfindungsgemäß beispielsweise eingesetzt:
Diethylamin, Triethylamin, Ethanolamin, Diethanolamin, Triethanolamin, n-oder iso-Propylamin, Di-n- oder Di-iso-propylamin, Tri-n- oder Tri-iso-propylamin, n-, iso- oder tert.-Butylamin, Amylamin, Hexylamin, Cyclohexylamin, Dicyclohexylamin, Stearylamin, Oleylamin, Talgfettamin, Cocosalkylamin, Distearylamin, Dimethylstearylamin und Dimethylbenzylamin.

Von den Aminen der Formel (IIb) werden erfindungsgemäß beispielsweise eingesetzt:
Ethylendiamin, Propylendiamin, Diethylentriamin, Dipropylentriamin, Triethylentetramin, Tripropylentetramin, 1,4-Diaminobutan, 1,5-Diaminopentan und 1,6-Diaminohexan.

Von den Aminen der Formel (IIc) werden erfindungsgemäß beispielsweise eingesetzt:
Pyrrolidin, Piperidin, Piperazin, Hexahydro-s-triazin, Urotropin, Hexamethylenimin, oder Trimethylenimin.

Es kann auch eine Mischung aus mindestens zwei der vorstehend genannten Amine eingesetzt werden.

Von den vorstehend beschriebenen Alkali- und Erdalkaliverbindungen werden erfindungsgemäß insbesondere eingesetzt:
Natriumhydrogencarbonat, Natriumcarbonat, Kaliumcarbonat, Dinatriumhydrogenphosphat, Dikaliumhydrogenphosphat, Calciumhydroxid, Natriumhydroxid, Kaliumhydroxid, Natriumethanolat, Kaliumethanolat, Natriummethanolat, Kaliummethanolat, Natriumpropanolat oder eine Mischung dieser Basen.

Die Basen werden erfindungsgemäß in Mengen von 0,01 bis 10 Gew.-%, vorzugsweise 0,1 bis 2 Gew.-%, bezogen auf das Aminobenzotrifluorid, eingesetzt. Mengen über 10 Gew.-% der genannten Basen sind ebenfalls zur Stabilisierung der Aminobenzotrifluoride geeignet, aber aus ökologischen und ökonomischen Gesichtspunkten nicht mehr zweckmäßig.

Als Aminobenzotrifluoride der Formel (I) sind beispielsweise von Interesse:

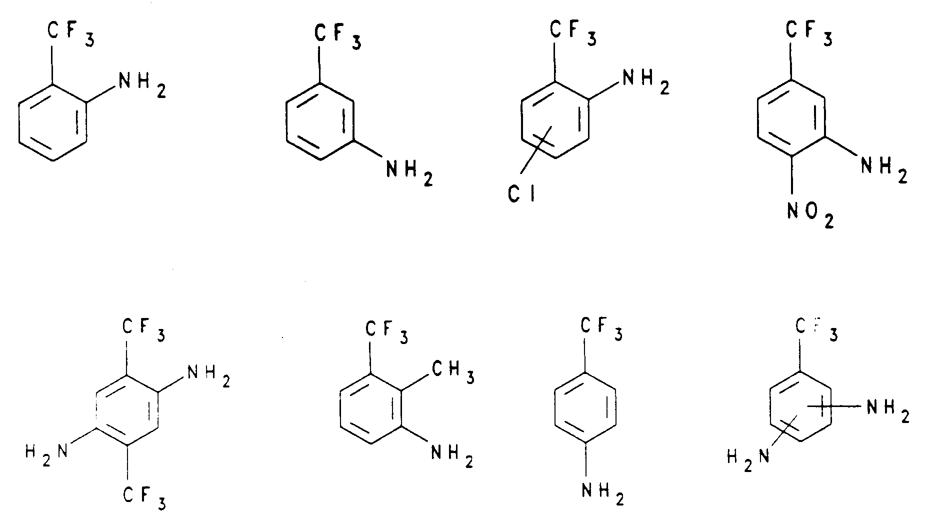

Von besonderem technischen Interesse sind 2-Aminobenzotrifluorid, 4-Aminobenzotrifluorid und 2-Amino-5-chlor-benzotrifluorid.

Gegenstand der vorliegenden Erfindung ist weiterhin ein Verfahren zur Stabilisierung von Aminobenzotrifluoriden der vorstehend definierten Formel (I), dadurch gekennzeichnet, daß man mindestens eine der vorstehend genannten Basen dem Aminobenzotrifluorid zusetzt, vorzugsweise vor oder während einer thermischen Belastung, beispielsweise einer Destillation. Die Basen können während einer thermischen Belastung auch kontinuierlich oder nach Bedarf in Portionen zugegeben werden. Ferner können die Basen zur Erhöhung der Lagerstabilität dem rohen oder gereinigten Aminobenzotrifluorid zugesetzt werden.

Der stabilisierende Einfluß der genannten Basen auf die Aminobenzotrifluoride ist sehr hoch. So läßt sich beispielsweise 2-Aminobenzotrifluorid bei Zusatz von 0,5 Gew.-% einer Base mehrere Tage auf 150 bis 160°C erhitzen, ohne daß Zersetzung unter Harzbildung eintritt. Durch gaschromatographische Analyse lassen sich keine Zersetzungsprodukte feststellen. 4-Aminobenzotrifluorid bleibt bei Zusatz einer Base über 5 Stunden bei 150°C stabil, während es ohne Zusatz bereits nach 1,5 Stunden bei 120°C verharzt. Besonders geeignet zur Erhöhung der Lagerstabilität bei höheren Temperaturen sind Amine. Diese lösen sich in den Aminobenzotrifluoriden, so daß ein ständiges Rühren oder Umwälzen der Mischung unnötig ist.

Destilliert man 2-Aminobenzotrifluorid über vorstehend genannte Basen, so ist das Destillat stabiler als ohne Basenzusatz erhaltene Destillate.

Gegenstand der vorliegenden Erfindung ist daher auch die Verwendung der vorstehend genannten Basen zur Erhöhung der Lagerstabilität sowie der Thermostabilität von Aminobenzotrifluoriden der Formel (I).

Die nachfolgenden Beispiele erläutern das erfindungsgemäße Verfahren, ohne es darauf zu beschränken.

### Beispiele

### 1) Thermostabilität von 2-Aminobenzotrifluorid (Vergleichsbeispiel)

10,0 g (62,1 mmol) 2-Aminobenzotrifluorid wurden unter Stickstoffatmosphäre auf 150°C erhitzt. Nach etwa 1,5 Stunden beobachtete man, daß das Edukt gelb und zähflüssig wurde und nach etwa 2 Stunden war es vollständig verharzt und erstarrt. Im gebildeten Harz ließen sich durch GC-, GC-MS- und MS-Analysen neben geringen Mengen an Edukt etwa 40 % verdampfbare Anteile mit Massen größer als 700 g/mol sowie unverdampfbare Komponenten mit hohen Molmassen nachweisen.

### 2) Thermostabilität von 4-Aminobenzotrifluorid (Vergleichsbeispiel)

5,0 g (31 mmol) 4-Aminobenzotrifluorid wurden unter Stickstoffatmosphäre auf 120°C erhitzt. Nach etwa 1,5 Stunden war das Edukt vollständig verharzt.

### 3) Thermostabilität von 2-Amino-5-chlor-benzotrifluorid (Vergleichsbeispiel)

10,0 g (51,2 mmol) 2-Amino-5-chlor-benzotrifluorid wurden unter Stickstoffatmosphäre auf 180°C erhitzt. Nach etwa 3 Stunden war das Edukt vollständig verharzt.

Die nachfolgenden Beispiele 4 bis 21 wurden mit gleichem Ergebnis mit und ohne Stickstoffüberlagerung durchgeführt.

### 4) Thermostabilität von 2-Aminobenzotrifluorid nach Zusatz von NaHCO₃

25,0 g (155 mmol) 2-Aminobenzotrifluorid wurden unter Zusatz von 0,5 g NaHCO₃ 56 Stunden auf 150°C erhitzt. Es wurde keine Verharzung beobachtet. Zersetzungsprodukte ließen sich mittels GC nicht nachweisen.

### 5) Thermostabilität von 2-Amino-5-chlor-benzotrifluorid nach Zusatz von NaHCO₃

25,0 g (128 mmol) 2-Amino-5-chlor-benzotrifluorid wurden unter Zusatz von 0,5 g NaHCO₃ 56 Stunden auf 150°C erhitzt. Es wurde keine Verharzung beobachtet. Zersetzungsprodukte ließen sich mittels GC nicht nachweisen.

### Beispiele 6 bis 18:

### Thermostabilität von 2-Aminobenzotrifluorid nach Zusatz von verschiedenen Basen

Die in der nachfolgenden Tabelle aufgeführten Beispiele 6 bis 18 wurden analog zu Beispiel 4 mit jeweils 100 g (0,62 mol) 2-Aminobenzotrifluorid durchgeführt und bei einer Temperatur von 150°C jeweils 8 Stunden erhitzt.

| Beispiel | Base |
|---|---|
| 6 | 0,5 g NaHCO₃ |
| 7 | 0,5 g Na₂CO₃ |
| 8 | 0,5 g NaOH (fest) |
| 9 | 0,5 g Na₂HPO₄ |
| 10 | 0,5 g Ca(OH)₂ |
| 11 | 0,5 g Stearylamin |
| 12 | 0,5 g Dimethylbenzylamin |
| 13 | 0,5 g Triethylamin |
| 14 | 0,5 g Cyclohexylamin |
| 15 | 0,5 g Urotropin |
| 16 | 0,5 g Dimethylstearylamin |
| 17 | 0,5 g Natriummethanolat |
| 18 | 0,5 g Distearylamin |

In allen aufgeführten Beispielen blieb das 2-Aminobenzotrifluorid stabil. Es wurde keine Verharzung oder Zersetzung beobachtet.

### 19) Thermostabilität von 4-Aminobenzotrifluorid nach Zusatz von Stearylamin

10,0 g (62,1 mmol) 4-Aminobenzotrifluorid wurden unter Zusatz von 0,5 g Stearylamin auf 150°C erhitzt. Das 4-Aminobenzotrifluorid blieb über 5 Stunden stabil.

### 20) Thermostabilität von 2-Amino-5-chlor-benzotrifluorid nach Zusatz von Stearylamin

100 g (0,52 mol) 2-Amino-5-chlor-benzotrifluorid wurden unter Zusatz von 0,5 g Stearylamin auf 185°C erhitzt. Das 2-Amino-5-chlor-benzotrifluorid blieb über 8 Stunden stabil. Es wurde keine Zersetzung oder Verharzung beobachtet.

### 21) Thermostabilität von 4-Aminobenzotrifluorid nach Zusatz von NaHCO₃

10,0 g (62,1 mmol) 4-Aminobenzotrifluorid wurden unter Zusatz von 0,5 g NaHCO₃ auf 150°C erhitzt. Das 4-Aminobenzotrifluorid blieb über 5 Stunden stabil.

### 22) Destillation von 2-Aminobenzotrifluorid unter Zusatz von Stearylamin

100,0 g (621 mmol) 2-Aminobenzotrifluorid wurden mit 0,5 g Stearylamin gemischt und bei 80°C/0,04 bar über eine 20 cm Vigreux-Kolonne destilliert. Das so erhaltene Destillat wird auf 150°C erhitzt. Erst nach 6 Stunden gab es Anzeichen einer beginnenden Verharzung, nach etwa 7,5 Stunden war der Ansatz vollständig verharzt.

## Patentansprüche

1. Präparation eines stabilisierten Aminobenzotrifluorids, bestehend im wesentlichen aus
a) einem Aminobenzotrifluorid der Formel (I), 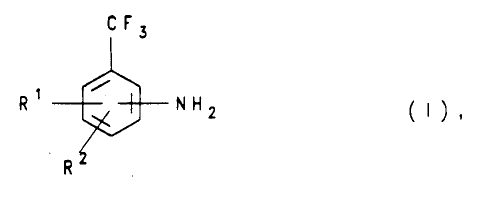 worin
R¹ und R² unabhängig voneinander Wasserstoff, C₁-C₆-Alkyl, C₂-C₆-Alkenyl, substituiertes oder unsubstituiertes Aryl, Halogen, CF₃, Nitro, Amino, Methoxy, Hydroxy oder NR³R⁴ bedeuten, wobei R³ und R⁴ unabhängig voneinander für Wasserstoff oder C₁-C₅-Alkylreste stehen, und
die NH₂-Gruppe in 2-, 3- oder 4-Position zur CF₃-Gruppe stehen kann und
b) mindestens einer Base.

2. Präparation gemäß Anspruch 1, dadurch gekennzeichnet, daß im Aminobenzotrifluorid der Formel (I)
R¹ und R² unabhängig voneinander jeweils für Wasserstoff, C₁-C₄-Alkyl, vorzugsweise Methyl, C₂-C₄-Alkenyl, Fluor, Chlor, Brom, CF₃, NO₂, NH₂, OCH₃, OH, substituiertes oder unsubstituiertes Phenyl, vorzugsweise Phenyl, Hydroxyphenyl, Tolyl, Xylyl, Aminophenyl, Halogenphenyl oder Nitrophenyl, oder NR³R⁴, worin R³ und R⁴ unabhängig voneinander Wasserstoff oder C₁-C₃-Alkyl bedeuten, stehen.

3. Präparation gemäß Anspruch 1 oder 2, dadurch gekennzeichnet, daß das Aminobenzotrifluorid 2-Aminobenzotrifluorid, 3-Aminobenzotrifluorid, 4-Aminobenzotrifluorid, 2-Amino-5-chlor-benzotrifluorid, 3-Amino-4-nitrobenzotrifluorid , 2,5-Diamino-4-trifluormethyl-benzotrifluorid, 3-Amino-2-methylbenzotrifluorid oder ein Diaminobenzotrifluorid ist.

4. Präparation nach mindestens einem der Ansprüche 1 bis 3, dadurch gekennzeichnet, daß die Base ein primäres, sekundäres oder tertiäres Amin der Formeln (IIa) oder (IIb) oder ein cycloaliphatisches Amin der Formel (IIc) ist
R⁵R⁶R⁷N (IIa),
R⁸-[-NR⁹-(CR¹³R¹⁴)ₙ-]ₘ-NR¹⁰R¹¹ (IIb), 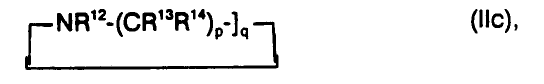
worin
R⁵, R⁶ und R⁷ unabhängig voneinander jeweils für Wasserstoff, C₁-C₄₀-Alkyl, vorzugsweise C₁-C₂₀-Alkyl, C₃-C₈-Cycloalkyl, vorzugsweise C₅-C₆-Cycloalkyl, CY¹Y²-Aryl, wobei Y¹ und Y² unabhängig voneinander C₁-C₄-Alkyl, Aryl oder Wasserstoff bedeuten und Aryl vorzugsweise für substituiertes oder unsubstituiertes Phenyl, insbesondere für Phenyl, Aminophenyl, Hydroxyphenyl, Tolyl, Xylyl, Nitrophenyl oder Halogenphenyl, steht,
R⁸, R⁹, R¹⁰, R¹¹ und R¹² jeweils unabhängig voneinander für Wasserstoff, C₁-C₂₀-Alkyl oder C₃-C₈-Cycloalkyl, vorzugsweise für Wasserstoff oder C₁-C₃-Alkyl, stehen, wobei R¹² zusätzlich die Bedeutung -(CR¹³R¹⁴)ₚ- haben kann,
R¹³ und R¹⁴ unabhängig voneinander für Wasserstoff oder Methyl stehen,
m für eine ganze Zahl von 1 bis 5, vorzugsweise 1 bis 2,
n für eine ganze Zahl von 2 bis 10, vorzugsweise 2 bis 3,
p für eine ganze Zahl von 1 bis 8, vorzugsweise 1 bis 6, und
q für eine ganze Zahl von 1 bis 5, vorzugsweise 1 bis 4, stehen,
oder eine Mischung der vorstehend genannten Amine ist.

5. Präparation nach mindestens einem der Ansprüche 1 bis 4, dadurch gekennzeichnet, daß die Base Stearylamin, Diethylamin, Triethylamin, Cyclohexylamin, Dimethylbenzylamin, Ethanolamin, Diethanolamin, Triethanolamin, n- oder iso-Propylamin, Di-n- oder Di-iso-propylamin, Tri-n- oder Tri-iso-propylamin, n-, iso- oder tert.-Butylamin, Amylamin, Hexylamin, Cyclohexylamin, Oleylamin, Talgfettamin, Cocosalkylamin, Distearylamin, Dimethylstearylamin, Dimethylbenzylamin, Ethylendiamin, Propylendiamin, Diethylentriamin, Dipropylentriamin, Triethylentetramin, Tripropylentetramin, 1,4-Diaminobutan, 1,5-Diaminopentan, 1,6-Diaminohexan, Pyrrolidin, Piperidin, Piperazin, Hexahydro-s-triazin, Urotropin, Hexamethylenimin, Trimethylenimin oder eine Mischung aus mindestens zwei der genannten Amine ist.

6. Präparation nach mindestens einem der Ansprüche 1 bis 5, dadurch gekennzeichnet, daß die Base Stearylamin, Triethylamin, Cyclohexylamin, Dimethylstearylamin, Distearylamin, Urotropin oder eine Mischung aus mindestens zwei der genannten Amine ist.

7. Präparation nach mindestens einem der Ansprüche 1 bis 3, dadurch gekennzeichnet, daß die Base ein Alkalicarbonat, Erdalkalicarbonat, Alkalihydrogencarbonat, Alkalihydrogenphosphat, Erdalkalihydrogenphosphat, Alkalialkoholat, Erdalkalialkoholat, Alkalihydroxid oder Erdalkalihydroxid ist, wobei Alkali vorzugsweise für Lithium, Natrium oder Kalium und Erdalkali vorzugsweise für Magnesium oder Calcium stehen, oder eine Mischung der vorstehend genannten Basen.

8. Präparation nach Anspruch 7, dadurch gekennzeichnet, daß die Base Natriumhydrogencarbonat, Natriumcarbonat, Natriumhydroxid, Natriummethanolat, Dinatriumhydrogenphosphat, Calciumhydroxid oder eine Mischung aus mindestens zwei der genannten Basen ist.

9. Präparation nach mindestens einem der Ansprüche 1 bis 8, dadurch gekennzeichnet, daß die Base in einer Menge von 0,01 bis 10 Gew.-%, vorzugsweise 0,1 bis 2 Gew.-%, bezogen auf das Aminobenzotrifluorid, enthalten ist.

10. Verfahren zur Stabilisierung von Aminobenzotrifluoriden der Formel (I) nach mindestens einem der Ansprüche 1 bis 3, dadurch gekennzeichnet, daß man dem Aminobenzotrifluorid mindestens eine Base zusetzt.

11. Verfahren nach Anspruch 10, dadurch gekennzeichnet, daß man die Base vor oder während einer thermischen Belastung dem Aminobenzotrifluorid zusetzt.

12. Verwendung der Basen nach mindestens einem der Ansprüche 4 bis 9 zur Erhöhung der Lagerstabilität und der Thermostabilität von Aminobenzotrifluoriden der Formel (I) nach mindestens einem der Ansprüche 1 bis 3.

## Claims

1. A preparation of a stabilized aminobenzotrifluoride, composed essentially of
a) an aminobenzotrifluoride of the formula (I), 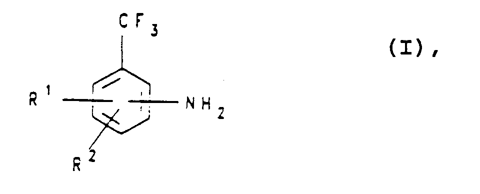 in which
R¹ and R², independently of each other, are hydrogen, C₁-C₆-alkyl, C₂-C₆-alkenyl, substituted or unsubstituted aryl, halogen, CF₃, nitro, amino, methoxy, hydroxyl or NR³R⁴, where R³ and R⁴, independently of each other, are hydrogen or C₁-C₅-alkyl radicals, and
the NH₂ group can be located in the 2-, 3- or 4-position in relation to the CF₃ group, and
b) at least one base.

2. The preparation as claimed in claim 1, wherein, in the aminobenzotrifluoride of the formula (I),
R¹ and R², independently of each other, are in each case hydrogen, C₁-C₄-alkyl, preferably methyl, C₂-C₄-alkenyl, fluorine, chlorine, bromine, CF₃, NO₂, NH₂, OCH₃, OH, substituted or unsubstituted phenyl, preferably phenyl, hydroxyphenyl, tolyl, xylyl, aminophenyl, halophenyl or nitrophenyl, or NR³R⁴, in which R³ and R⁴, independently of each other, are hydrogen or C₁-C₃-alkyl.

3. The preparation as claimed in claim 1 or 2, wherein the aminobenzotrifluoride is 2-aminobenzotrifluoride, 3-aminobenzotrifluoride, 4-aminobenzotrifluoride, 2-amino-5-chlorobenzotrifluoride, 3-amino-4-nitrobenzotrifluoride, 2,5-diamino-4-trifluoromethylbenzotrifluoride, 3-amino-2-methylbenzotrifluoride or a diaminobenzotrifluoride.

4. The preparation as claimed in at least one of claims 1 to 3, wherein the base is a primary, secondary or tertiary amine of the formulae (IIa) or (IIb) or a cycloaliphatic amine of the formula (IIc)
R⁵R⁶R⁷N (IIa),
R⁸-[-NR⁹-(CR¹³R¹⁴)ₙ-]ₘ-NR¹⁰R¹¹ (IIb), 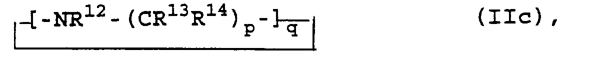
in which
R⁵, R⁶ and R⁷, independently of each other, are in each case hydrogen, C₁-C₄₀-alkyl, preferably C₁-C₂₀-alkyl, C₃-C₈-cycloalkyl, preferably C₅-C₆-cycloalkyl or CY¹Y²-aryl, where Y¹ and Y², independently of each other, are C₁-C₄-alkyl, aryl or hydrogen, and aryl is preferably substituted or unsubstituted phenyl, in particular phenyl, aminophenyl, hydroxyphenyl, tolyl, xylyl, nitrophenyl or halophenyl,
R⁸, R⁹, R¹⁰, R¹¹ and R¹², in each case independently of each other, are hydrogen, C₁-C₂₀-alkyl or C₃-C₈-cycloalkyl, preferably hydrogen or C₁-C₃-alkyl, where R¹² can additionally have the meaning of -(CR¹³R¹⁴)ₚ-,
R¹³ and R¹⁴, independently of each other, are hydrogen or methyl,
m is an integer from 1 to 5, preferably 1 to 2,
n is an integer from 2 to 10, preferably 2 to 3,
p is an integer from 1 to 8, preferably 1 to 6, and
q is an integer from 1 to 5, preferably 1 to 4,
or is a mixture of the abovementioned amines.

5. The preparation as claimed in at least one of claims 1 to 4, wherein the base is stearylamine, diethylamine, triethylamine, cyclohexylamine, dimethylbenzylamine, ethanolamine, diethanolamine, triethanolamine, n- or iso-propylamine, di-n- or di-iso-propylamine, tri-n- or tri-iso-propylamine, n-, iso-or tert-butylamine, amylamine, hexylamine, cyclohexylamine, oleylamine, tallow fatty amine, coconut alkylamine, distearylamine, dimethylstearylamine, dimethylbenzylamine, ethylenediamine, propylenediamine, diethylenetriamine, dipropylenetriamine, triethylenetetramine, tripropylenetetramine, 1,4-diaminobutane, 1,5-diaminopentane, 1,6-diaminohexane, pyrrolidine, piperidine, piperazine, hexahydro-s-triazine, Urotropine, hexamethyleneimine, trimethyleneinine, or a mixture of at least two of said amines.

6. The preparation as claimed in at least one of claims 1 to 5, wherein the base is stearylamine, triethylamine, cyclohexylamine, dimethylstearylamine, distearylamine, Urotropine, or a mixture of at least two of said amines.

7. The preparation as claimed in at least one of claims 1 to 3, wherein the base is an alkali metal carbonate, alkaline earth metal carbonate, alkali metal hydrogen carbonate, alkali metal hydrogen phosphate, alkaline earth metal hydrogen phosphate, alkali metal alcoholate, alkaline earth metal alcoholate, alkali metal hydroxide or alkaline earth metal hydroxide, where alkali metal is preferably lithium, sodium or potassium and alkaline earth metal is preferably magnesium or calcium, or a mixture of the abovementioned bases.

8. The preparation as claimed in claim 7, wherein the base is sodium hydrogen carbonate, sodium carbonate, sodium hydroxide, sodium methanolate, disodium hydrogen phosphate, calcium hydroxide, or a mixture of at least two of said bases.

9. The preparation as claimed in at least one of claims 1 to 8, wherein the base is contained in a quantity of 0.01 to 10% by weight, preferably 0.1 to 2% by weight, based on the aminobenzotrifluoride.

10. A process for stabilizing aminobenzotrifluorides of the formula (I) as claimed in at least one of claims 1 to 3, wherein at least one base is added to the aminobenzotrifluoride.

11. The process as claimed in claim 10, wherein the base is added to the aminobenzotrifluoride before or during a thermal stress.

12. The use of the bases as claimed in at least one of claims 4 to 9 for increasing the storage stability and the thermal stability of aminobenzotrifluorides of the formula (I) as claimed in at least one of claims 1 to 3.

## Revendications

1. Préparation d'un aminobenzotrifluorure stabilisé composé essentiellement
a) d'un aminobenzotrifluorure de formule (I), 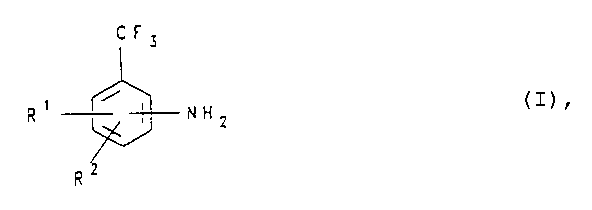 dans laquelle
R¹ et R², indépendamment l'un de l'autre, représentent l'hydrogène, alkyle en C₁-C₆, alcényle en C₂-C₆, aryle substitué ou non substitué, halogène, CF₃, nitro, amino, méthoxy, hydroxy ou -NR³R⁴, R³ et R⁴, indépendamment l'un de l'autre, étant l'hydrogène ou des radicaux alkyle en C₁-C₅, et
le groupe NH₂ pouvant être en position 2, 3 ou 4 par rapport au groupe CF₃ et
b) d'au moins une base.

2. Préparation selon la revendication 1, caractérisée en ce que dans l'aminobenzotrifluorure de formule (I)
R¹ et R², indépendamment l'un de l'autre, représentent chaque fois l'hydrogène, alkyle en C₁-C₄, de préférence méthyle, alcényle en C₂-C₄, le fluor, le chlore, le brome, CF₃, NO₂, NH₂, OCH₃, OH, phényle substitué ou non substitué, de préférence phényle, hydroxyphényle, tolyle, xylyle, aminophényle, halogénophényle ou nitrophényle ou NR³R⁴, où R³ et R⁴, indépendamment l'un de l'autre, représentent l'hydrogène ou alkyle en C₁-C₃.

3. Préparation selon la revendication 1 ou 2, caractérisée ce que l'aminobenzotrifluorure est 2-aminobenzotrifluorure, 3-aminobenzotrifluorure, 4-aminobenzotrifluorure, 2-amino-5-chloro-benzotrifluorure, 3-amino-4-nitro-benzotrifluorure, 2,5-diamino-4-trifluorométhyl-benzotrifluorure, 3-amino-2-méthyl-benzotrifluorure ou un diaminobenzo-trifluorure.

4. Préparation selon au moins l'une des revendications 1 à 3, caractérisée ce que la base est une amine primaire, secondaire ou tertiaire, de formules (IIa) ou (IIb) ou une amine cycloaliphatique de formule (IIc)
R⁵R⁶R⁷N (IIa),
R⁸-[-NR⁹-(CR¹³R¹⁴)ₙ-]ₘ-NR¹⁰R¹¹ (IIb), 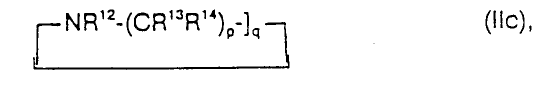
où
R⁵, R⁶ et R⁷, indépendamment l'un de l'autre, représentent chacun l'hydrogène, alkyle en C₁-C₄₀, de préférence alkyle en C₁-C₂₀, cycloalkyle en C₃-C₈, de préférence cycloalkyle en C₅-C₆, CY¹Y²-aryle, Y¹ et Y², indépendamment l'un de l'autre, représentant alkyle en C₁-C₄, aryle ou l'hydrogène et aryle de préférence représentant phényle substitué ou non substitué, plus particulièrement phényle, aminophényle, hydroxyphényle, tolyle, xylyle, nitrophényle et halogénophényle,
R⁸, R⁹, R¹⁰, R¹¹ et R¹², indépendamment l'un de l'autre, représentent chacun l'hydrogène, alkyle en C₁-C₂₀ ou cycloalkyle en C₃-C₈, de préférence l'hydrogène ou alkyle en C₁-C₃, R¹² pouvant avoir de plus la signification -(CR¹³R¹⁴)ₚ-,
R¹³ et R¹⁴, indépendamment l'un de l'autre, représentent l'hydrogène ou méthyle,
m est un nombre entier de 1 à 5, de préférence de 1 à 2,
n est un nombre entier de 2 à 10, de préférence de 2 à 3,
p est un nombre entier de 1 à 8, de préférence de 1 à 6, et
q est un nombre entier de 1 à 5, de préférence de 1 à 4,
ou un mélange des amines précitées.

5. Préparation selon au moins l'une des revendications 1 à 4, caractérisée ce que la base est stéarylamine, diéthylamine, triéthylamine, cyclohexylamine, diméthylbenzylamine, éthanolamine, diéthanolamine, triéthanolamine, n- ou iso-propylamine, di-n- ou di-isopropylamine, tri-n- ou tri-iso-propylamine, n-, iso- ou tert-butylamine, amylamine, hexylamine, cyclohexylamine, oléylamine, amine de suif, cocosalkylamine, distéarylamine, diméthylstéarylamine, diméthylbenzylamine, éthylènediamine, propylènediamine, diéthylènetriamine, dipropylènetriamine, triéthylènetatramine, tripropylènetétramine, 1,4-diaminobutane, 1,5-diaminopentane, 1,6-diaminohexane, pyrrolidine, pipéridine, pipérazine, hexahydro-s-triazine, urotropine, hexaméthylèneimine, triéthylèneimine ou un mélange d'au moins deux des amines précitées.

6. Préparation selon au moins l'une des revendications 1 à 5, caractérisée ce que la base est stéarylamine, triéthylamine, cyclohexylamine, diméthylstéarylamine, distéarylamine, urotropine ou un mélange d'au moins deux des amines précitées.

7. Préparation selon au moins l'une des revendications 1 à 3, caractérisée ce que la base est un carbonate de métaux alcalins, carbonate de métaux alcalino-terreux, bicarbonate de métaux alcalins, hydrogénophosphate de métaux alcalins, hydrogénophosphate de métaux alcalino-terreux, alcoolate de métaux alcalins, alcoolate de métaux alcalino-terreux, hydroxyde de métaux alcalins ou hydroxyde de métaux alcalino-terreux, alcali étant de préférence le lithium, le sodium ou le potassium et le métal alcalino-terreux étant de préférence le magnésium ou le calcium, ou un mélange des bases précitées.

8. Préparation selon la revendication 7, caractérisée ce que la base est bicarbonate de sodium, carbonate de sodium, hydroxyde de sodium, méthanolate de sodium, hydrogénophosphate disodique, hydroxyde de calcium ou un mélange d'un moins deux des bases précitées.

9. Préparation selon au moins l'une des revendications 1 à 8, caractérisée ce que la base est contenue dans une quantité de 0,01 à 10 % en poids, de préférence de 0,1 à 2 % en poids par rapport à l'aminobenzotrifluorure.

10. Procédé pour la stabilisation des aminobenzotrifluorures de formule (I) selon au moins l'une des revendications 1 à 3, caractérisé ce que l'on ajoute à l'aminobenzotrifluorure au moins une base.

11. Procédé selon la revendication 10, caractérisé en ce qu'on ajoute la base avant ou pendant la charge thermique de l'aminobenzotrifluorure.

12. Utilisation des bases selon au moins l'une des revendications 4 à 9 pour augmenter la stabilité au stockage et la stabilité thermique des aminobenzotrifluorures de formule (I), selon au moins l'une des revendications 1 à 3.
